# EUROPEAN PATENT APPLICATION

(11) **EP 1 921 547 A2**
(43) Date of publication of application: **14.05.2008**
(21) Application number: 07018418.9
(22) Date of filing: 19.09.2007
(51) Int. Cl.: G06F 17/20

(54) **Image display apparatus**

(30) Priority: 09.11.2006 JP 2006304522
(71) Applicant: OLYMPUS MEDICAL SYSTEMS CORP., Shibuya-ku Tokyo 151-0072 (JP)
(72) Inventor: Oda, Yasuharu c/o OLYMPUS MEDICAL SYSTEMS CORPORATION, Tokyo 151-0072 (JP)
(74) Representative: von Hellfeld, Axel

(57) **Abstract**

An image display apparatus (4) according to the present invention includes a display unit (12) that displays a series of intra-subject images capturing an inside of a subject (1) along time series, and additionally displays a thumbnail corresponding to an intra-subject image selected from the series of intra-subject images; a grouping processor (16b) that creates a thumbnail group including a plurality of thumbnails; an input unit (11) that inputs a thumbnail comment about the thumbnail; and a comment processor (16c) that appends the thumbnail comment to the thumbnail, combines a plurality of thumbnail comments respectively of the plurality of thumbnails included in the thumbnail group whenever the grouping processor (16b) creates the thumbnail group, and creates a group comment of the thumbnail group.

## Description

### CROSS-REFERENCE TO RELATED APPLICATIONS

This application is based upon and claims the benefit of priority from Japanese Patent Application No. 2006-304522, filed on Nov. 9, 2006, the entire contents of which are incorporated herein by reference.

### BACKGROUND OF THE INVENTION

### 1. Field of the Invention

The present invention relates to an image display apparatus which displays a series of images, of an inside of a subject body, captured in time series.

### 2. Description of the Related Art

In recent years, a capsule endoscope including an imaging function and a wireless communication function has been developed in the field of an endoscope. The capsule endoscope is swallowed from a mouth into a body of a subject for an observation (examination), and travels inside of organs of the subject body such as the stomach and the small intestine according to their peristalsis until it is naturally excreted to capture images of the inside of the organs sequentially at predetermined intervals, hereinbelow the images occasionally being referred to as intra-subject images. The capsule endoscope wirelessly transmits the infra-subject images sequentially to a receiver carried by the subject (see Japanese Patent Application Laid-Open No. 2003-19111).

The infra-subject images wirelessly transmitted from the capsule endoscope are sequentially received by the receiver, and stored in a storage medium of the receiver. Then, the storage medium storing the intra-subject images of the subject is detached from the receiver, and attached to an image display apparatus. The image display apparatus acquires the intra-subject images via the storage medium, and displays the intra-subject images on a display. A doctor, a nurse, or the like makes a diagnosis of the subject body via the observation of the intra-subject images displayed on the image display apparatus.

When a user selects a desired intra-subject image from the intra-subject images displayed on the display, the image display apparatus additionally displays a thumbnail image corresponding to the selected intra-subject image. When the user performs an input operation for inputting a desired comment (hereinafter referred to as thumbnail comment) with respect to the displayed thumbnail image, the image display apparatus appends a thumbnail comment input via the input operation to the corresponding thumbnail image. The image display apparatus, by repeating such a processing, can append desired thumbnail comment to each of a plurality of thumbnails.

The thumbnail comment, for example, indicates a reason for having selected the intra-subject image corresponding to the thumbnail image, i.e., the desired intra-subject image selected, by the user, from the intra-subject images displayed on the display. The user can easily recognize what the intra-subject image corresponding to the thumbnail image is like, by visually checking the thumbnail comment.

The image display apparatus includes a grouping function in which the plurality of thumbnails are grouped. In this case, the image display apparatus creates a group including a desired group of thumbnails selected by the user from the plurality of thumbnails (hereinafter referred to as thumbnail group). The thumbnail group is, for example, a collection of thumbnails whose thumbnail comments are of the same kind in content (the reason for having selected the intra-subject image as described above, for example) with each other.

However, when the user does not perform an input operation for inputting a desired comment with respect to the thumbnail group (hereinafter referred to as group comment), the conventional image display apparatus described above cannot append the group comment to the thumbnail group. For this reason, whenever the convention image display apparatus creates the thumbnail group, the user has to perform the input operation repetitively for inputting the group comment for each thumbnail group even if the group comment is substantially no more than the thumbnail comment of each thumbnail in the thumbnail group. This is the reason why the conventional image display apparatus has a problem that the operation in creating the group comment with respect to the thumbnail group is cumbersome and complicated.

### SUMMARY OF THE INVENTION

It is an object of the present invention to at least solve the described problems in the conventional technology.

An image display apparatus according to one aspect of the present invention includes a display unit that displays a series of intra-subject images capturing an inside of a subject along time series, and additionally displays a thumbnail corresponding to an intra-subject image selected from the series of infra-subject images; a grouping processor that creates a thumbnail group including a plurality of thumbnails; an input unit that inputs a thumbnail comment about the thumbnail; and a comment processor that appends the thumbnail comment to the thumbnail, combines a plurality of thumbnail comments respectively of the plurality of thumbnails included in the thumbnail group whenever the grouping processor creates the thumbnail group, and creates a group comment of the thumbnail group.

The above and other objects, features, advantages and technical and industrial significance of the present invention will be better understood by reading the following detailed description of presently preferred embodiments of the invention, when considered in connection with the accompanying drawings.

### BRIEF DESCRIPTION OF THE DRAWINGS

FIG. 1 is a schematic view of a configuration example of an intra-subject information acquiring system including an image display apparatus according to an embodiment of the present invention;
FIG. 2 is a schematic block diagram of a configuration example of the image display apparatus according to the embodiment of the present invention;
FIG. 3 is a flowchart of a processing procedure of a comment processor in creating a group comment;
FIG. 4 is a schematic view of an example of a window displayed on a display unit when the comment processor creates the group comment;
FIG. 5 is a schematic view explaining an operation of the comment processor which combines a plurality of thumbnail comments to create the group comment;
FIG. 6 is a schematic view explaining an operation of the comment processor which combines a plurality of group comments to create a new group comment;
FIG. 7 is a schematic view explaining an operation of the comment processor which divides a group comment of a thumbnail group whose grouping is canceled;
FIG. 8 is a schematic view of an example of a window displayed on the display unit when intra-subject images of a subject and the like are displayed;
FIG. 9 is a schematic view of an example of a window displayed on the display unit when various kinds of processing are executed on at least one thumbnail displayed in a subimage display area;
FIG. 10 is a schematic view of an example of a window displayed on the display unit in inputting various kinds of information such as a diagnosis result and the like of the subject to be inserted to a diagnosis report;
FIG. 11 is a schematic view of an example of a window displayed on the display unit in checking various kinds of information such as a thumbnail, a thumbnail comment, and the like to be inserted to the diagnosis report; and
FIG. 12 is a schematic view of an example of a window displaying the diagnosis report of the subject.

### DETAILED DESCRIPTION OF THE PREFERRED EMBODIMENTS

Exemplary embodiments of an image display apparatus according to the present invention will be explained in detail below with reference to the accompanying drawings. It should be noted that the invention is not limited by the embodiments.

### Embodiment

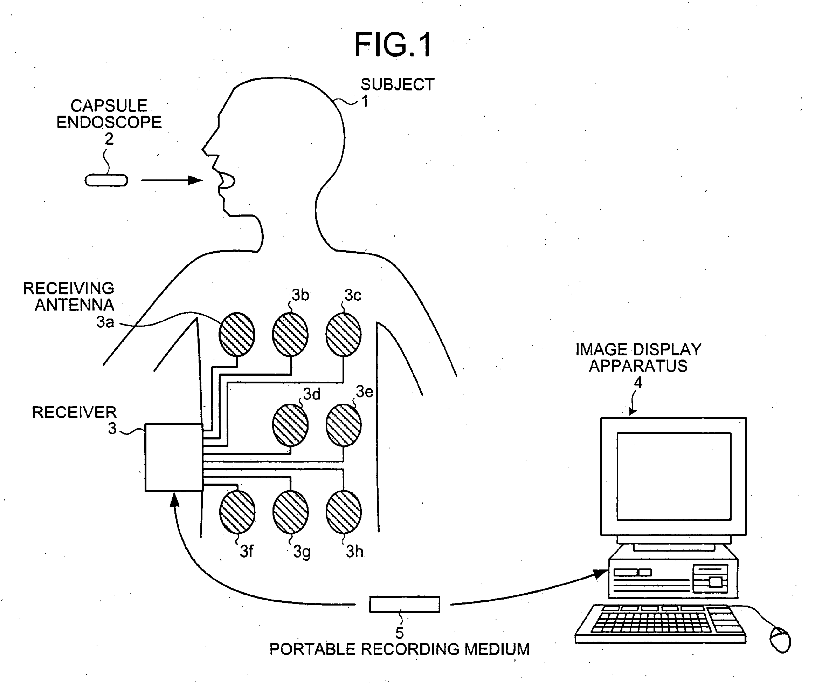

FIG. 1 is a schematic view of a configuration example of an intra-subject information acquiring system including an image display apparatus according to an embodiment of the present invention. As shown in FIG. 1, the intra-subject information acquiring system includes a capsule endoscope 2 which captures intra-subject images of a subject 1; a receiver 3 which receives the intra-subject images of the subject 1 from the capsule endoscope 2; an image display apparatus 4 which displays the intra-subject images of the subject 1 received by the receiver, i.e., the intra-subject images captured by the capsule endoscope 2; and a portable recording medium 5 which transfers data between the receiver 3 and the image display apparatus 4.

The capsule endoscope 2 captures intra-subject images of the subject 1, and has an imaging function and a wireless communication function inside a capsule chassis which can be inserted into the inside of organs of the subject 1. Specifically, the capsule endoscope 2, after swallowed from a mouth of the subject 1, travels inside of the organs of the subject 1 according to their peristalsis to capture intra-subject images sequentially at predetermined intervals (at an interval of 0.5 second, for example). Then, the capsule endoscope 2 wirelessly transmits image signals including the intra-subject images sequentially to the receiver 3 placed outside of the subject 1.

The receiver 3 receives the intra-subject images of the subject 1 captured by the capsule endoscope 2. Specifically, the receiver 3 includes a plurality of receiving antennas 3a to 3h, and is attached to (carried by) the subject 1 of which the capsule endoscope 2 is inserted to the inside of the organs. The receiver 3 sequentially receives, via the receiving antennas 3a to 3h, image signals wirelessly transmitted from the capsule endoscope 2 inside the organs of the subject 1, and acquires the intra-subject images captured by the capsule endoscope 2. The receiver 3 has the portable recording medium 5 to store the intra-subject images of the subject 1 received from the capsule endoscope 2 therein.

The receiving antennas 3a to 3h are placed to disperse on a body surface of the subject 1 along the traveling path of the capsule endoscope 2 inserted to the inside of the organs of the subject 1, i.e., along the alimentary canal of the subject 1, and connected to the receiver 3. The receiving antennas 3a to 3h catch image signals wirelessly transmitted sequentially from the capsule endoscope 2 in the inside of the organs of the subject 1, and transmit the caught image signals sequentially to the receiver 3. The receiving antennas 3a to 3h may be placed to disperse on a jacket and the like to be worn by the subject 1. The number of antennas for acquiring the image signals from the capsule endoscope 2 is not specifically limited to eight as long as at least one antenna is placed with respect to the subject 1.

The image display apparatus 4 has a configuration like a workstation in which various data such as the intra-subject images of the subject 1 is acquired via the portable recording medium 5, and displayed on a display. The image display apparatus 4 displays the intra-subject images of the subject 1 acquired via the portable recording medium 5. A user such as a doctor and a nurse observes (examines) the intra-subject images of the subject 1 displayed in the image display apparatus 4 to make a diagnosis on the subject 1. The image display apparatus 4 has a report creating function for creating a diagnosis report which shows a diagnosis result and the like of the subject 1, and displays the created diagnosis report on the display.

The portable recording medium 5, being a portable type of recording medium, transfers data between the receiver 3 and the image display apparatus 4. Specifically, the portable recording medium 5 is detachable with respect to the receiver 3 and the image display apparatus 4, having a configuration capable of outputting and recording data when attached thereto. The portable recording medium 5 when attached to the receiver 3, records the intra-subject images and the like of the subject 1 which the receiver 3 has received from the capsule endoscope 2, and when attached to the image display apparatus 4, outputs the recorded data such as the intra-subject images and the like of the subject 1 to the image display apparatus 4.

The various data recorded in the portable recording medium 5 is, for example, the intra-subject images of the subject 1, temporal information (imaging time, reception time, and the like) of respective intra-subject images contained in the intra-subject images, patient information of the subject 1, examination information of the subject 1, and the like. Here, the patient information of the subject 1 is specific information which specifies the patient 1, for example, the patient name, ID, birth date, sex, age, and the like of the subject 1. The examination information of the subject 1 is specific information which specifies the capsule endoscopic examination (an examination for observing the inside of the organs with the capsule endoscope 2 inserted inside of the organs) to be performed on the subject 1, for example, ID, date, and the like of the examination.

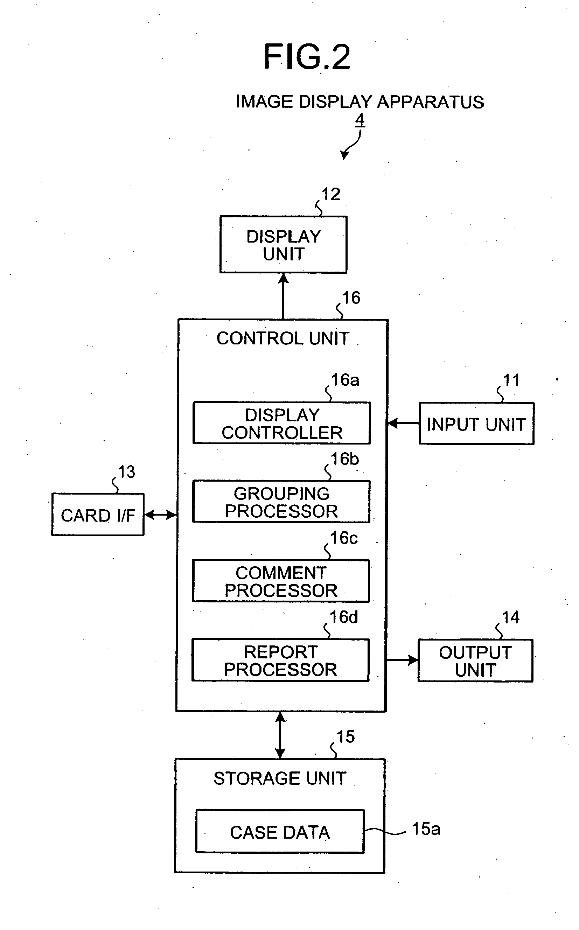

Next, a configuration of the image display apparatus 4 according to the embodiment of the present invention will be explained. FIG. 2 is a schematic block diagram of a configuration example of the image display apparatus according to the embodiment of the present invention. As shown in FIG. 2, the image display apparatus 4 according to this embodiment includes an input unit 11 that inputs various kinds of information; a display unit 12 that displays the intra-subject images of the subject 1; and a card interface (I/F) 13 which takes various data stored in the portable recording medium 5 therein. The image display apparatus 4 further includes an output unit 14 which outputs the diagnosis report and the like of the subject 1; a storage unit 15 which stores various data such as the intra-subject images and the like of the subject 1; and a control unit 16 which controls components of the image display apparatus 4.

The input unit 11 is realized by using an input device such as a keyboard and a mouse. The input unit 11 inputs various kinds of information to the control unit 16 based on an input operation performed by the user. Such various kinds of information input by the input unit 11 to the control unit 16 is, for example, instruction information which gives an instruction to the control unit 16, patient information of the subject 1, examination information of the subject 1, a thumbnail comment about a thumbnail displayed on the display unit 12, and the like.

The display unit 12 realized by using various types of display devices such as a CRT display and a liquid crystal display, displays various kinds of information which is instructed, by the control unit 16, to be displayed. Specifically, the display unit 12 displays the intra-subject images of the subject 1 captured by the capsule endoscope 2, a reduced-size image which is simply visualized from a desired intra-subject image selected by the user from the intra-subject images (for example, a thumbnail), patient information of the subject 1, examination information of the subject 1, the diagnosis report of the subject 1, and the like.

The card I/F 13, to which the portable recording medium 5 is detachably attached, inputs and outputs data between the attached portable recording medium 5 and the control unit 16. Specifically, the card I/F 13 reads data recorded in the attached portable recording medium 5 (for example, the intra-subject images of the subject 1, temporal information of respective intra-subject images, patient information and examination information of the subject 1, and the like), and sends the read data to the control unit 16. In addition, the card I/F 13 writes various data, which the control unit 16 instructs to write into the portable recording medium 5 (for example, patient information and examination information of the subject 1, and the like), into the portable recording medium 5.

The output unit 14, for example realized by using a printer and the like, prints and outputs various data, which the control unit 16 instructs to output, to a medium such as paper. Specifically, the output unit 14 prints various data such as the infra-subject images, the diagnosis report, and the like of the subject 1 to the medium such as paper, and outputs the medium to which various data is printed.

The storage unit 15 is realized by using various types of storage media such as the random access memory (RAM), electrically erasable and programmable read only memory (EEPROM), flash memory, or a hard disc, and the like, which rewritably store data. The storage unit 15 stores various data, which the control unit 16 instructs to store, and sends data which the control unit 16 instructs to read among the stored various data to the control unit 16. The storage unit 15 stores case data 15a of plural subjects therein. Case datum of one subject (the subject 1, for example) contained in the case data 15a includes intra-subject images, patient information, examination information, temporal information of respective intra-subject images, diagnosis report information, and the like of the subject.

The storage unit 15 may be realized by using a drive which can detachably attach a portable recording medium such as a flexible disc (FD), compact disc (CD), digital versatile disc (DVD), and the like, and performs a reading processing or a writing processing of various data on the attached portable recording medium.

The control unit 16 controls components (the input unit 11, the display unit 12, the card I/F 13, the output unit 14, and the storage unit 15) of the image display apparatus 4 and controls an input and an output of signals between those components. Specifically, the control unit 16 controls the card I/F 13 to acquire data recorded in the portable recording medium 5, and controls the storage unit 15 to store the acquired data as one piece of the case data 15a. The control unit 16 reads, based on instruction information input by the input unit 11, case datum of a desired subject among the case data 15a, and controls the display unit 12 to display the intra-subject images and the like of the subject which are contained in the read case datum. Further, the control unit 16 controls, based on instruction information input by the input unit 11, the output unit 14 to output data such as the intra-subject images, diagnosis report, and the like of the subject.

The control unit 16 includes a display controller 16a, a grouping processor 16b, a comment processor 16c, and a report processor 16d. The display controller 16a controls the display unit 12 to display a graphical user interface (GUI) of various kinds for making a diagnosis on the subject. The display controller 16a further controls, based on instruction information input by the input unit 11, the display unit 12 to display various data. Various data displayed on the display unit 12 according to the control of the display controller 16a includes, for example, various kinds of information input by the input unit 11, intra-subject images of the subject 1 selected among the case data 15a, at least one thumbnail corresponding to at least one intra-subject image selected from the intra-subject images, a thumbnail comment about the thumbnail, at least one thumbnail group which has a plurality of thumbnails, a group comment about the thumbnail group, a diagnosis report, and the like of the subject 1.

The grouping processor 16b functions as a group creating unit that creates the thumbnail group including a plurality of thumbnails. The grouping processor 16b, based on instruction information input by the input unit 11, groups a plurality of thumbnails to create at least one thumbnail group including plural thumbnails.

Further, the grouping processor 16b has an ungrouping function for ungrouping a thumbnail group based on instruction information input by the input unit 11. The groping processor 16b excludes at least one thumbnail designated by the user from the thumbnail group designated to be ungrouped by the user, and ungroups the designated thumbnail group. In this case, the grouping processor 16b excludes at least one thumbnail from the designated thumbnail group, and changes the designated thumbnail group to another thumbnail group. Otherwise, the grouping processor 16b excludes a plurality of thumbnails from the designated thumbnail group, changes the designated thumbnail group to another thumbnail group, and also creates a new thumbnail group which includes the plurality of thumbnails excluded from the designated thumbnail group.

The comment processor 16c functions as a comment processing unit that creates a group comment about the thumbnail group created by the grouping processor 16b. Specifically, the comment processor 16c appends, in a state where a desired thumbnail is selected (designated) by the user among the thumbnails displayed on the display unit 12, a thumbnail comment input by the input unit 11 to the thumbnail designated by the user. The comment processor 16c repeats such a processing, and uniquely associates at least one thumbnail comment with at least one thumbnail, respectively. When the grouping processor 16b creates a thumbnail group, the comment processor 16c combines a plurality of thumbnail comments respectively corresponding to a plurality of thumbnails included in the thumbnail group, and creates a group comment about the thumbnail group.

When the grouping processor 16b excludes at least one thumbnail from the designated thumbnail group and ungroups the designated thumbnail group, the comment processor 16c deletes a thumbnail comment about the excluded at least one thumbnail from the group comment about the designated thumbnail group. Then, the comment processor 16c changes the group comment of the designated thumbnail group to a thumbnail comment of another thumbnail group. Further the comment processor 16c appends at least one thumbnail comment which is deleted from the group comment of the designated thumbnail group to at least one thumbnail excluded from the designated thumbnail group. Otherwise, the comment processor 16c combines respective thumbnail comments of a plurality of thumbnails excluded from the designated thumbnail group, and creates a group comment about a new thumbnail group including the plurality of thumbnails.

The report processor 16d functions as a report creating unit that creates a diagnosis report of the subject 1 based on the case datum of the subject 1 selected from the case data 15a. The report processor 16d creates a diagnosis report in which, for example, the thumbnail group created by the grouping processor 16b, the group comment generated by the comment processor 16c about the thumbnail group, patient information of the subject 1, and the like are inserted. In this case, the group comment is inserted into the diagnosis report as an observation result (findings found by the user) of the intra-subject images of the subject 1 which correspond to the plurality of thumbnails included in the thumbnail group.

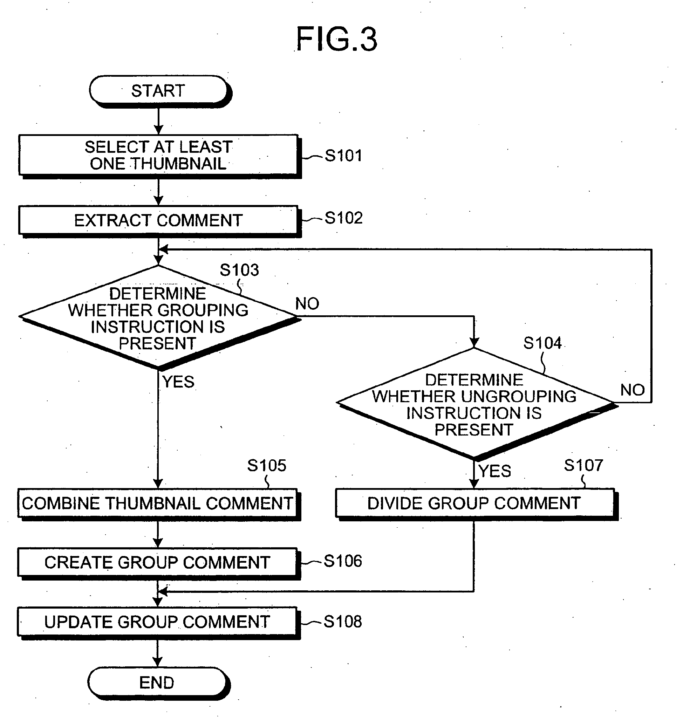

Next, a processing procedure of the comment processor 16c which creates the group comment as described above will be explained. FIG. 3 is a flowchart of the processing procedure followed by the comment processor 16c in creating the group comment. As described, the comment processor 16c uniquely associates at least one thumbnail comment input by the input unit 11 with at least one thumbnail designated by the user respectively, and uses a plurality of thumbnail comments associated with a plurality of thumbnails, respectively to create a group comment.

More specifically, as shown in FIG. 3, the comment processor 16c selects at least one thumbnail designated by the user among the thumbnails displayed on the display unit 12 (step S101). In this case, the comment processor 16c selects, based on instruction information input by the input unit 11, at least one thumbnail designated by the user. The at least one thumbnail is a thumbnail designated by the user for creating a thumbnail group, or a thumbnail to be excluded from an existing thumbnail group for ungrouping the existing thumbnail group.

Next, the comment processor 16c extracts a comment such as the thumbnail comment about at least one thumbnail selected at step S101 (step S102). Specifically, the comment processor 16c extracts at least one thumbnail comment which is appended to at least one thumbnail designated by the user at step S101. Here, when at least one thumbnail is in the existing thumbnail group, the comment processor 16c also extracts a group comment about the existing thumbnail group.

Then, the comment processor 16c determines whether a grouping instruction which instructs to create a thumbnail group is present (step S103). At step S103, when no grouping instruction is input by the input unit 11, the comment processor 16c determines that no grouping instruction is present ("No" at step 5103), and determines whether an ungrouping instruction which instructs to ungroup the existing thumbnail group is present (step 5104). At step S104, when no ungrouping instruction is input by the input unit 11, the comment processor 16c determines that no ungrouping instruction is present ("No" at step S104.) In this case, the comment processor 16c returns to step S103 and repeats the processing procedure from step S103. The comment processor 16c may determine whether the ungrouping instruction is present after performing the processing procedure of step S102, and determine whether the grouping instruction is present after determining that no ungrouping instruction is present.

On the other hand, when the grouping instruction is input by the input unit 11 at step S103, the comment processor 16c determines that the grouping instruction is present ("Yes" at step S103). In this case, the grouping processor 16b groups the plurality of thumbnails designated by the user at step S101, and creates a thumbnail group including the plurality of thumbnails. The comment processor 16c combines a plurality of thumbnail comments about the plurality of thumbnails included in the thumbnail group created by the grouping processor 16b (step S105), and thereby creates a group comment about the thumbnail group (step S106).

The comment processor 16c combines the plurality of thumbnail comments about the plurality of thumbnails extracted at step S102, and creates the group comment about the thumbnail group in performing the processing procedure of steps S105 and S106. In this case, the comment processor 16c puts a separator such as a space (blank), slash (/), and the like between respective thumbnail comments. Thus, the comment processor 16c combines the plurality of thumbnail comments so that respective thumbnail comments are discriminable to each other.

On the other hand, when the ungrouping instruction is input by the input unit 11 at step S104, the comment processor 16c determines that the ungrouping instruction is present ("Yes" at step S104). In this case the grouping processor 16b deletes at least one thumbnail designated by the user at step S101 from the existing thumbnail group (the thumbnail group designated, by the user, to be ungrouped), and ungroups the existing thumbnail group. The comment processor 16c divides the group comment of the existing thumbnail group ungrouped by the grouping processor 16b with respect to at least one thumbnail, or with respect to at least one thumbnail group (step S107).

Specifically, when the grouping processor 16b ungroups the existing thumbnail group by excluding at least one thumbnail from the existing thumbnail group, the comment processor 16c deletes the thumbnail comment, of at least one thumbnail excluded by the grouping processor 16b, i.e., at least one thumbnail as an exclusion target, from the group comment of the existing thumbnail group, changes the group comment of the existing thumbnail group to a group comment of another thumbnail group, and appends at least one thumbnail comment deleted from the group comment of the existing thumbnail group to at least one thumbnail as the exclusion target. In other words, the comment processor 16c divides the group comment of the existing thumbnail group into a group comment of another thumbnail group and a thumbnail comment of at least one thumbnail as the exclusion target.

Furthermore, when the grouping processor 16b excludes a plurality of thumbnails from the existing thumbnail group, changes the existing thumbnail group to another thumbnail group, and creates a new thumbnail group including the plurality of thumbnails, the comment processor 16c deletes a plurality of thumbnail comments of the plurality of thumbnails excluded by the grouping processor 16b from the group comment of the existing thumbnail group, changes the group comment of the existing thumbnail group to a group comment of another thumbnail group, and combines the thumbnail comments of the plurality of thumbnails similarly to step S105 to create the group comment of the new thumbnail group. In other words, the comment processor 16c divides the group comment of the existing thumbnail group into the group comment of another thumbnail group and the group comment of the new thumbnail group.

The comment processor 16c updates, after performing the processing procedure of step S106 or step S107, the group comment of the thumbnail group created or changed by the grouping processor 16b (step S108).

Specifically, when the group comment is created at step S106 or step S107 which is just before step S108, the comment processor 16c newly sets the created group comment as a group comment of the thumbnail group created by the grouping processor 16b. On the other hand, when the group comment is divided at step S107 which is just before step S108, the comment processor 16c updates the group comment of the existing thumbnail group ungrouped by the grouping processor 16b with the group comment after division. In this case, the group comment after the division updated by the comment processor 16c belongs to another thumbnail group formed by changing (dividing) the existing thumbnail group, and is stored in the storage unit 15 as one piece of the case data 15a.

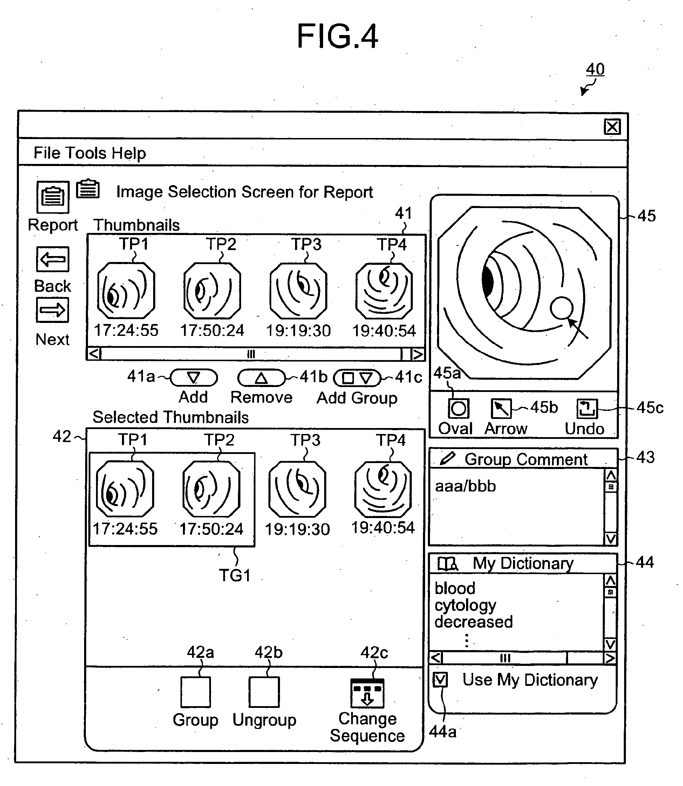

Next, a display mode of the display unit 12 when the comment processor 16c creates the group comment will be explained. FIG. 4 is a schematic view of an example of a window displayed on the display unit 12 when the comment processor 16c creates the group comment. As shown in FIG. 4, a window 40 has a GUI for performing various kinds of processing about thumbnails such as the grouping or ungrouping of thumbnails. The display condition of the window 40 is controlled by the display controller 16a.

Specifically, the window 40 includes a first thumbnail display area 41 which displays at least one thumbnail, a second thumbnail display area 42 which displays a desired thumbnail selected from at least one thumbnail displayed in the first thumbnail display area 41, and a comment box 43 for displaying and creating the thumbnail comment and the group comment. The window 40 further includes an "Add" icon 41a, "Remove" icon 41b, and an "Add Group" icon 41c adjacent to the first thumbnail display area 41, and includes a "Group" icon 42a, an "Ungroup" icon 42b, and a "Change Sequence" icon 42c adjacent to the second thumbnail display area 42. Moreover, the window 40 includes a dictionary 44 for inputting existing character information in the comment box 43, and a marker appending area 45 for appending a predetermined mark to thumbnails displayed in the first thumbnail display area 41 or the second thumbnail display area 42.

The first thumbnail display area 41 displays at least one thumbnail corresponding to at least one intra-subject image selected by the user from the intra-subject images of the subject 1. For example as shown in FIG. 4, four thumbnails TP1 to TP4 are displayed in the first thumbnail display area 41. The first thumbnail display area 41 has a function of scrolling the thumbnails and can display desired number of thumbnails, not limiting to the four thumbnails TP1 to TP4.

The "Add" icon 41a is an icon for additionally displaying, in the second thumbnail display area 42, at least one thumbnail selected by the user from at least one thumbnail displayed in the first thumbnail display area 41. Specifically, when the user selects the thumbnail TP1 from the thumbnails TP1 to TP4 displayed in the first thumbnail display area 41 and clicks on the "Add" icon 41a, the selected thumbnail TP1 is additionally displayed in the second thumbnail display area 42. When the user selects a plurality of thumbnails from the thumbnails TP1 to TP4 displayed in the first thumbnail display area 41 and clicks on the "Add" icon 41a, the plurality of thumbnails are collectively displayed in the second thumbnail display area 42.

The "Remove" icon 41b is an icon for returning at least one thumbnail selected by the user from at least one thumbnail additionally displayed in the second thumbnail display area 42 back to the first thumbnail display area 41, i.e., for removing from the second thumbnail display area 42. Specifically, when the user selects the thumbnail TP1 from the thumbnails TP1 to TP4 displayed in the second thumbnail display area 42 and clicks on the "Remove" icon 41b, the selected thumbnail TP1 is removed from the second thumbnail display area 42 and returned back to the first thumbnail display area 41. When the user selects a plurality of thumbnails from the thumbnails TP1 to TP4 displayed in the second thumbnail display area 42 and clicks on the "Remove" icon 41b, the plurality of thumbnails are collectively deleted from the second thumbnail display area 42 and returned back to the first thumbnail display area 41.

The "Add Group" icon 41c is an icon for grouping a plurality of thumbnails selected by the user from at least one thumbnail displayed in the first thumbnail display area 41 and additionally displaying the grouped thumbnails in the second thumbnail display area 42. Specifically, when the user selects a plurality of thumbnails TP1 and TP2 from the thumbnails TP1 to TP4 displayed in the first thumbnail display area 41 and clicks on the "Add Group" icon 41c, the input unit 11 inputs information of instructing to group the selected thumbnails TP1 and TP2 to the control unit 16. In this case, the grouping processor 16b creates a thumbnail group TG1 including the plurality of thumbnails TP1 and TP2. The thumbnail group TG1 created by the grouping processor 16b is additionally displayed in the second thumbnail display area 42 so that the plurality of thumbnails TP1 and TP2 can be visually recognized. The comment processor 16c combines thumbnail comments respectively of the plurality of thumbnails TP1 and TP2 and creates a group comment of the thumbnail group TG1.

The second thumbnail display area 42 additionally displays at least one thumbnail selected by the user from at least one thumbnail displayed in the first thumbnail display area 41. For example as shown in FIG. 4, the second thumbnail display area 42 displays the four thumbnails TP1 to TP4 selected from the first thumbnail display area 41. When the thumbnails TP1 and TP2 are grouped by the grouping processor 16b from the four thumbnails TP1 to TP4, the second thumbnail display area 42 displays the thumbnail group TG1 so that the plurality of thumbnails TP1 and TP2 can be visually recognized.

The "Group" icon 42a is an icon for grouping a plurality of thumbnails displayed in the second thumbnail display area 42. Specifically, when the user selects the thumbnails TP1 and TP2 from the thumbnails TP1 to TP4 displayed in the second thumbnail display area 42 and clicks on the "Group" icon 42a, the input unit 11 inputs information of instructing to group the selected thumbnails TP1 and TP2 to the control unit 16. In this case, the grouping processor 16b creates the thumbnail group TG1 including the plurality of thumbnails TP1 and TP2. The thumbnail group TG1 created by the grouping processor 16b is displayed in the second thumbnail display area 42 so that the plurality of thumbnails TP1 and TP2 can be visually recognized. The comment processor 16c combines thumbnail comments respectively of the plurality of thumbnails TP1 and TP2, and creates the group comment of the thumbnail group TG1.

The "Ungroup" icon 42b is an icon for ungrouping the thumbnail group displayed in the second thumbnail display area 42. Specifically, when the user selects the thumbnail TP1 in the thumbnail group TG1 displayed in the second thumbnail display area 42 and clicks on the "Ungroup" icon 42b, the input unit 11 inputs information of instructing to ungroup the thumbnail group TG1 including the selected thumbnail TP1 to the control unit 16. In this case, the grouping processor 16b excludes the selected thumbnail TP1 from the thumbnail group TG1, and ungroups the thumbnail group TG1. The second thumbnail display area 42 displays the thumbnails TP1 and TP2 which are ungrouped (divided) from the thumbnail group TG1 by the grouping processor 16b. The comment processor 16c divides the group comment of the ungrouped thumbnail group TG1 to the thumbnail TP1 and the thumbnail TP2.

The "Change Sequence" icon 42c is an icon for changing the sequence of the thumbnails or thumbnail groups displayed in the second thumbnail display area 42. Specifically, when the user selects a plurality of thumbnails displayed in the second thumbnail display area 42 and clicks on the "Change Sequence" icon 42c, the sequence of the plurality of thumbnails changes. Further, when the user selects a plurality of thumbnail groups displayed in the second thumbnail display area 42 and clicks on the "Change Sequence" icon 42c, the sequence of the plurality of thumbnail groups changes.

The comment box 43 is a text box for displaying and creating a thumbnail comment of the thumbnail or a group comment of the thumbnail group displayed in the first thumbnail display area 41 or the second thumbnail display area 42. Specifically, when the user selects a desired thumbnail from at least one thumbnail displayed in the first thumbnail display area 41 or the second thumbnail display area 42, the comment box 43 displays the thumbnail comment of the selected thumbnail in text data format. Further, when the user selects a desired thumbnail group ' from at least one thumbnail group displayed in the first thumbnail display area 41 or the second thumbnail display area 42, the comment box 43 displays the group comment of the selected thumbnail group, (the group comment "aaa/bbb" of the thumbnail group TG1, for example) in text data format. The thumbnail comment or the group comment displayed in the comment box 43 can be changed to a desired thumbnail comment or a group comment by operating the input unit 11. In this case, the comment processor 16c updates the thumbnail comment or the group comment with the text data input in the comment box 43.

The dictionary 44 includes preset plural pieces of text data, and functions as a dictionary unit which retrieves and displays a candidate of text data to be displayed in the comment box 43 from the plural pieces of text data. Specifically, when a desired character such as an alphabet is input in the comment box 43, the dictionary 44 retrieves at least one piece of text data whose initial is the input character from the preset plural pieces of text data, and displays the retrieval result. Then, the dictionary 44 displays text data selected by the user from at least one piece of text data displayed as the retrieval result in the comment box 43. For example when a character "b" is input in the comment box 43, the dictionary 44 displays text data "blood" whose initial is the character "b" as a candidate. When the user selects the text data "blood", the dictionary 44 displays the selected text data "blood" in the comment box 43. The dictionary function of the dictionary 44 can be switched over between a valid state and an invalid state via clicking on a check box 44a.

The marker appending area 45 is a GUI for appending a desired mark to the thumbnail displayed in the first thumbnail display area 41 or the second thumbnail display area 42. Specifically, the marker appending area 45 includes a marker icon 45a for appending a maker having a circle shape or an oval shape, and a marker icon 45b for appending a marker having an arrow shape. As shown in FIG. 4, the marker appending area 45 displays a thumbnail selected by the user from at least one thumbnail displayed in the first thumbnail display area 41 or the second thumbnail display area 42. When the marker icon 45a is clicked, the maker having a circle shape or an oval shape is appended on the displayed thumbnail in the marker appending area 45. When the marker icon 45b is clicked, the marker having an arrow shape is appended on the displayed thumbnail in the marker appending area 45.

The marker appending area 45 further includes an "Undo" icon 45c for cancelling the marker appended on the displayed thumbnail. When the "Undo" icon 45c is clicked, various markers, i.e., markers of a circle shape, an oval shape or an arrow shape, appended on the thumbnail are deleted in the marker appending area 45.

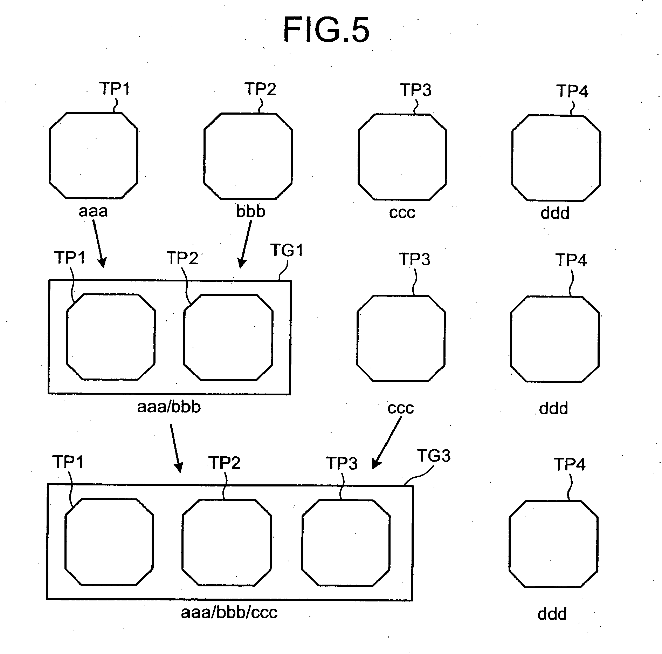

Next, an operation of the comment processor 16c in creating a group comment by combining a plurality of thumbnail comments will be explained specifically. FIG. 5 is a schematic view explaining the operation of the comment processor 16c which combines a plurality of thumbnail comments to create the group comment.

As shown in FIG. 5, when the grouping processor 16b groups the thumbnails TP1 and TP2 from the four thumbnails TP1 to TP4 (see FIG. 4), and creates the thumbnail group TG1, the comment processor 16c combines a thumbnail comment "aaa" of the thumbnail TP1 and a thumbnail comment "bbb" of the thumbnail TP2, and creates a group comment of the thumbnail group TG1 including the thumbnails TP1 and TP2. In this case, the comment processor 16c combines thumbnail comments "aaa" and "bbb" respectively of the thumbnails TP1 and TP2 according to the sequence of the thumbnails TP1 and TP2 in the thumbnail group TG1, and puts a predetermined separator "/" between the combined thumbnail comments "aaa" and "bbb". Thus, the comment processor 16c creates a group comment "aaa/bbb" of the thumbnail group TG1.

When the grouping processor 16b groups the thumbnail group TG1 and the thumbnail TP3, and creates a thumbnail group TG3, the comment processor 16c combines the group comment "aaa/bbb" of the thumbnail group TG1 and a thumbnail comment "ccc" of the thumbnail TP3, and creates a group comment of the thumbnail group TG3 including the thumbnails TP1, TP2, and TP3. In this case, the comment processor 16c the group comment "aaa/bbb" of the thumbnail group TG1 and the thumbnail comment "ccc" of the thumbnail TP3, and puts the predetermined separator "/" between the combined group comment "aaa/bbb" and the thumbnail comment "ccc". Thus, the comment processor 16c creates a group comment "aaa/bbb/ccc" of the thumbnail group TG3.

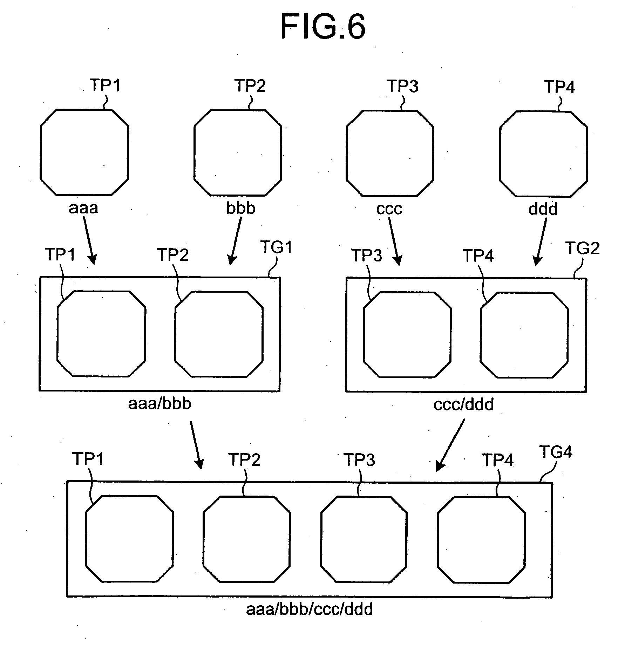

Next, an operation of the comment processor 16c in creating a new group comment by combining a plurality of group comments will be explained specifically. FIG. 6 is a schematic view explaining the operation of the comment processor 16c which combines a plurality of group comments to create a new group comment.

As shown in FIG. 6, when the grouping processor 16b creates the thumbnail group TG1, the comment processor 16c creates the group comment "aaa/bbb" of the thumbnail group TG1 as described above. Further, when the grouping processor 16b groups the thumbnails TP3 and TP4, and creates a thumbnail group TG2, the comment processor 16c combines the thumbnail comment "ccc" of the thumbnail TP3 and a thumbnail comment "ddd" of the thumbnail TP4, and creates a group comment "ccc/ddd" of the thumbnail group TG2 including the thumbnails TP3 and TP4, similarly to the case of creating the group comment of the thumbnail group TG1.

Here, when the grouping processor 16b groups a plurality of thumbnail groups TG1 and TG2, and creates a thumbnail group TG4, the comment processor 16c combines group comments "aaa/bbb" and "ccc/ddd" respectively of the thumbnail groups TG1 and TG2, and creates a group comment of the thumbnail group TG4 including the thumbnail groups TG1 and TG2. In this case, the comment processor 16c combines group comments "aaa/bbb" and "ccc/ddd" respectively of the thumbnail groups TG1 and TG2 according to the sequence of the thumbnail groups TG1 and TG2 in the thumbnail group TG4, i.e., the sequence of thumbnails TP1, TP2, TP3, and TP4 in the thumbnail group TG4, and puts the predetermined separator "/" between the combined group comments "aaa/bbb" and "ccc/ddd". Thus, the comment processor 16c creates a group comment "aaa/bbb/ccc/ddd" of the thumbnail group TG4.

When the thumbnail group TG4 is created via the grouping of the thumbnail group TG3 (see FIG. 5) and the thumbnail TP4, the comment processor 16c combines group comments "aaa/bbb/ccc" and "ddd" respectively of the thumbnail group TG3 and the thumbnail TP4 according to the sequence of the thumbnail group TG3 and the thumbnail TP4 in the thumbnail group TG4, and puts the predetermined separator "/" between the combined group comment "aaa/bbb/ccc" and the thumbnail comment "ddd". Thus, the comment processor 16c creates a group comment "aaa/bbb/ccc/ddd" of the thumbnail group TG4.

When the thumbnail group TG4 is created via the grouping of the thumbnail group TG1 and a plurality of thumbnails TP3 and TP4, the comment processor 16c combines the group comment "aaa/bbb" of the thumbnail group TG1, and thumbnail comments "ccc" and "ddd" respectively of the plurality of thumbnails TP3 and TP4 according to the sequence of the thumbnail group TG1, and the plurality of thumbnails TP3 and TP4 in the thumbnail group TG4, and puts the predetermined separator "/" between the group comment "aaa/bbb" and the thumbnail comment "ccc", and between the thumbnail comment "ccc" and the thumbnail comment "ddd". Thus, the comment processor 16c creates a group comment "aaa/bbb/ccc/ddd" of the thumbnail group TG4.

The comment processor 16c may put a desired separator for example ",", ".", "|", "-", and the like, except for the separator "/" described above, or may put a space (blank) between respective thumbnail comments which constitutes a group comment.

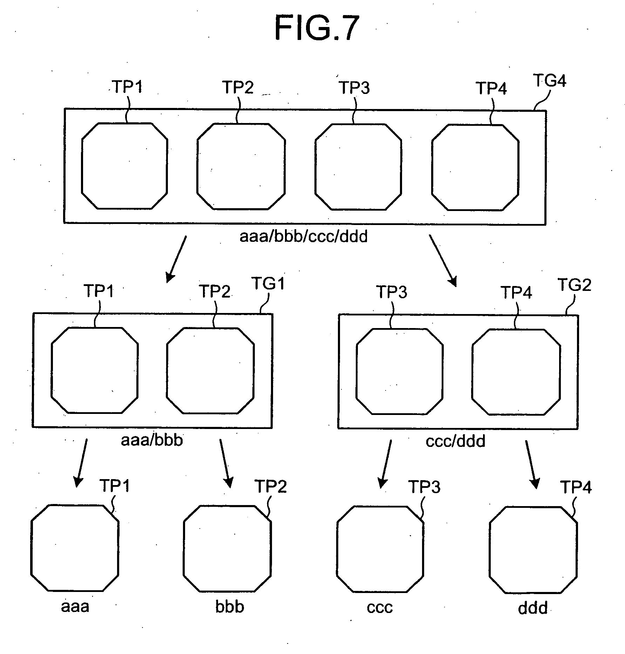

Next, an operation of the comment processor 16c in dividing a group comment of a thumbnail group which is ungrouped by the grouping processor 16b will be explained specifically. FIG. 7 is a schematic view explaining the operation of the comment processor 16c which divides a group comment of a thumbnail group whose grouping is canceled.

As shown in FIG. 7, when the thumbnail group TG4 is divided and ungrouped by the grouping processor 16b into the thumbnail group TG1 and the thumbnail group TG2, the comment processor 16c divides the group comment "aaa/bbb/ccc/ddd" of the thumbnail group TG4 to the thumbnail group TG1 and the thumbnail group TG2. In this case, the comment processor 16c divides the group comment "aaa/bbb/ccc/ddd" of the thumbnail group TG4, to append, with respect to the thumbnail group TG1, the comment portion "aaa/bbb" corresponding to the thumbnails TP1 and TP2 in the thumbnail group TG1, and to append, with respect to the thumbnail group TG2, the comment portion "ccc/ddd" corresponding to the thumbnails TP3 and TP4 in the thumbnail group TG2.

When the thumbnail group TG1 is divided'and ungrouped by the grouping processor 16b into a plurality of thumbnails TP1 and TP2, the comment processor 16c divides the group comment "aaa/bbb" of the thumbnail group TG1 to the thumbnail TP1 and the thumbnail TP2. In this case, the comment processor 16c divides the group comment "aaa/bbb" of the thumbnail group TG1, to append, with respect to the thumbnail TP1, the comment portion "aaa" corresponding to the thumbnail TP1, and to append, with respect to the thumbnail TP2, the comment portion "bbb" corresponding to the thumbnail TP2.

Similarly, when the thumbnail group TG2 is divided into a plurality of thumbnails TP3 and TP4, the comment processor 16c divides the group comment "ccc/ddd" of the thumbnail group TG2 to the thumbnail comment "ccc" of the thumbnail TP3 and the thumbnail comment "ddd" of the thumbnail TP4.

Further, when the grouping processor 16b excludes the thumbnail TP4 from the thumbnail group TG4, and changes the thumbnail group TG4 to another thumbnail group TG3 (see FIG. 5), the comment processor 16c deletes the thumbnail comment "ddd" of the excluded thumbnail TP4 from the group comment "aaa/bbb/ccc/ddd" of the thumbnail group TG4, creates the group comment "aaa/bbb/ccc" of the thumbnail group TG3, and appends the deleted thumbnail comment "ddd" to the thumbnail TP4.

When the grouping processor 16b excludes a plurality of thumbnails TP3 and TP4 from the thumbnail group TG4, and changes the thumbnail group TG4 to another thumbnail group TG1, the comment processor 16c deletes the thumbnail comments "ccc" and "ddd" respectively of the excluded thumbnails TP3 and TP4 from the group comment "aaa/bbb/ccc/ddd" of the thumbnail group TG4, creates the group comment "aaa/bbb" of the thumbnail group TG1, and appends the deleted plural thumbnail comments "ccc" and "ddd" to the plurality of thumbnails TP3 and TP4, respectively.

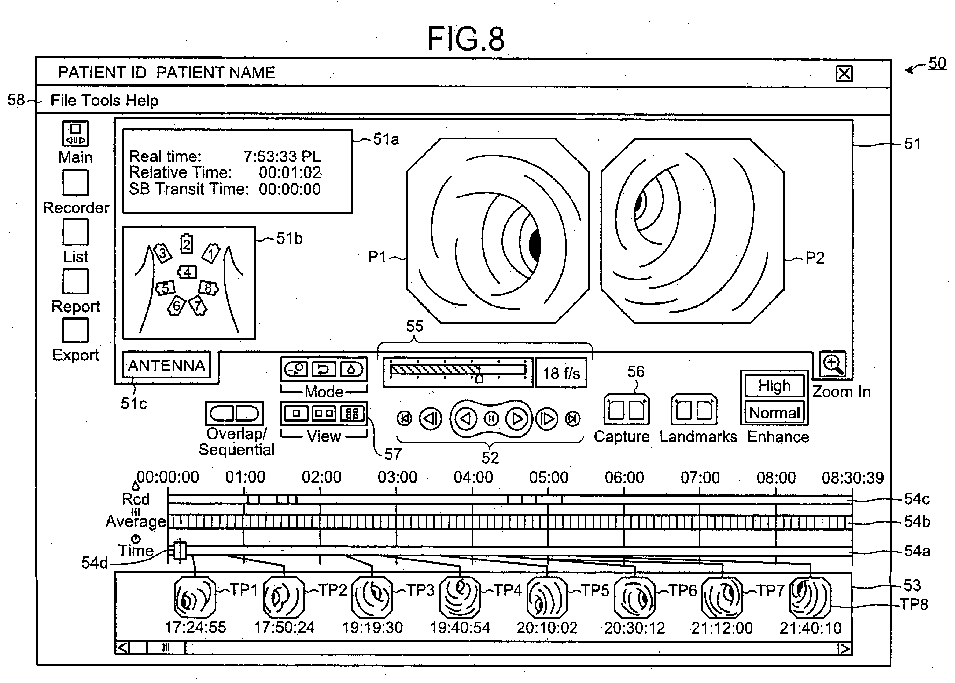

Next, a display mode of the display unit 12 in displaying intra-subject images included in the case datum of the subject 1 selected from the case data 15a will be explained. FIG. 8 is a schematic view of an example of a window displayed on the display unit 12 when the intra-subject images and the like of the subject 1 are displayed. When the control unit 16 performs a predetermined log-in processing, the display controller 16a controls the display unit 12 to display a window 50 as shown in FIG. 8. The window 50 has a GUI for displaying the intra-subject images of the subject 1 and observing the inside of organs of the subject 1. The display mode of the window 50 is controlled by the display controller 16a described above.

Specifically as shown in FIG. 8, the window 50 includes a main-image display area 51 which displays intra-subject images of the subject 1, display operation icons 52 which performs various display operations for displaying the intra-subject images of the subject 1 in the main- image display area 51, and a subimage display area 53 which displays a thumbnail corresponding to a desired intra-subject image selected from the intra-subject images of the subject 1. The window 50 further includes a time bar 54a which indicates a temporal position of each intra-subject image among the iritra-subject images of the subject 1, an average color bar 54b which displays an average color of the intra-subject images at each temporal position indicated by the time bar 54a, a bleed bar 54c which indicates a temporal position of at least one bleeding image in the intra-subject images of the subject 1, and a slider 54d which indicates a temporal position of an image which is currently displayed, among respective temporal positions indicated by the time bar 54a, in the main-image display area 51. The window 50 still further includes a frame rate display area 55 which displays a frame rate of the intra-subject images displayed in the main-image display area 51, a registration status display area 56 which displays information indicating whether or not the intra-subject images displayed in the main-image display area 51 are images whose thumbnails are registered, a display number setting area 57 in which the number of intra-subject images to be correctively displayed in the main-image display area 51 is set, and a menu bar 58 which displays various menus such as a file menu, a tool menu, and a help menu.

The main-image display area 51 sequentially displays the intra-subject images of the subject 1, in accordance with display operations (play, fast-forward, pause, and the like) using the display operation icons 52. The main-image display area 51 displays intra-subject images collectively in the number set in the display number setting area 57. For example, when "two" is set in the display number setting area 57 as the number of intra-subject images, the main-image display area 51 sequentially displays two intra-subject images of the subject 1 at once as exemplified with intra-subject images P1 and P2 shown in FIG. 8. The number of intra-subject images set by the display number setting area 57 is "one", "two", or "four", for example.

The main-image display area 51 has a temporal information display area 51a which displays temporal information of the intra-subject image which is currently displayed (hereinbelow referred to as "currently-displayed image"), and an antenna position information display area 51b. The temporal information display area 51a displays, for example, an imaging time of the currently-displayed image, a relative time of the currently-displayed image with respect to a head image, i.e., an intra-subject image whose imaging time is the earliest of all, and the like, as the temporal information of the currently-displayed image, exemplified as the intra-subject images P1 and P2 in FIG. 8, in the main-image display area 51.

The antenna position information display area 51b indicates a position of a receiving antenna which has caught the currently-displayed image with the highest receiving field intensity when the receiver 3 (see FIG. 1) has received the currently-displayed image via the receiving antennas 3a to 3h. The antenna position information display area 51b displays such antenna position information, so that an estimated position of the capsule endoscope when the currently-displayed image is captured can be indicated. The information displaying function of the antenna position information display area 51b can be switched over between a valid state and an invalid state via clicking on a display setting icon 51c.

The subimage display area 53 displays at least one thumbnail corresponding to at least one intra-subject image selected from the intra-subject images of the subject 1 displayed in the main-image display area 51. Specifically, whenever the user selects (clicks on) a desired intra-subject image from the intra-subject images sequentially displayed in the main-image display area 51 via the operation of the input unit 11, a thumbnail corresponding to the selected intra-subject image is registered. The subimage display area 53 sequentially adds and displays the registered thumbnail (thumbnails TP1 to TP8 in FIG. 8, for example). The subimage display area 53 has a scrolling function and can display a desired number of thumbnails, not limiting to the eight thumbnails TP1 to TP8.

When the window 40 (see FIG. 4) is displayed on the display unit 12, at least one thumbnail displayed in the subimage display area 53 is displayed in the first thumbnail display area 41 in the window 40.

The time bar 54a indicates a temporal position of each intra-subject image in the intra-subject images of the subject 1 displayed in the main-image display area 51. The slider 54d moves on the time bar 54a in accordance with the temporal information of the currently-displayed image in the main-image display area 51 and indicates the temporal position corresponding to the currently-displayed image on the time bar 54a. The average color bar 54b and the bleed bar 54c are formed along the temporal axis of the time bar 54a. The average color bar 54b displays, with respect to each temporal position, an average color calculated from a plurality of intra-subject images present at each temporal position of the time bar 54a. The bleed bar 54c, when the intra-subject images of the subject 1 includes at least one bleeding image (an intra-subject image capturing a bleeding region), indicates a temporal position of the bleeding image with a red marker, for example.

The frame rate display area 55 sequentially displays, with respect to each intra-subject image, a display speed (frame rate) in sequentially displaying the intra-subject images of the subject 1 in the main-image display area 51. Specifically, the frame rate display area 55 sequentially displays a frame rate of the currently-displayed image in the main-image display area 51 whenever at least one intra-subject image is displayed in the main-image display area 51. The frame rate display area 55 displays a level of the frame rate for the currently-displayed image in the main-image display area 51 by using a bar and a numeric value, for example as shown in FIG. 8. With the bar and the numeric value displayed in the frame rate display area 55, the user can easily and visually check whether or not the frame rate of at least one intra-subject image displayed in the main-image display area 51 is set to a desired level.

The registration status display area 56 displays information which indicates whether or not the thumbnail corresponding to the currently-displayed image in the main-image display area 51 is already registered. Specifically, the registration status display area 56 displays a predetermined mark (a mark having a triangular shape shown in FIG. 8, for example) in the number equivalent to the number of intra-subject images displayed at once in the main-image display area 51. Then, the registration status display area 56 changes a display mode of the predetermined mark (a display color, for example) so as to display the information indicating whether or not the thumbnail corresponding to the currently-displayed image is already registered. For example in FIG. 8, when the registration status display area 56 makes the color of the triangular mark on the left side blue, and the color of the triangular mark on the right side white, the intra-subject image P1 on the left side is an intra-subject image whose thumbnail is already registered, and the intra-subject image P2 on the right side is an intra-subject image whose thumbnail is not registered yet, out of the intra-subject images P1 and P2 each as the currently-displayed image in the main-image display area 51.

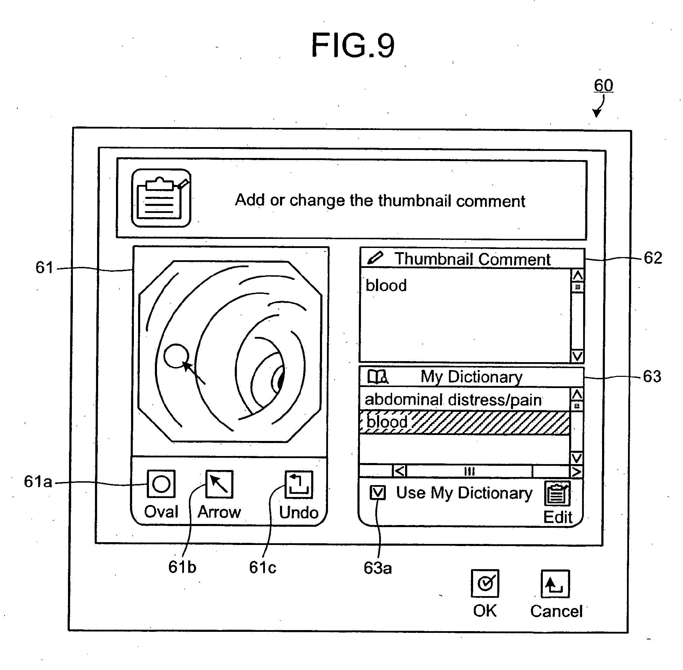

Next, a display mode of the display unit 12 in performing various kinds of processing such as a processing for appending a thumbnail comment, and a processing for appending a marker, with respect to at least one thumbnail displayed in the subimage display area 53 in the window 50 will be explained. FIG. 9 is a schematic view of an example of a window displayed on the display unit 12 when various kinds of processing are executed on at least one thumbnail displayed in the subimage display area 53 in the window 50. As shown in FIG. 9, a window 60 has a GUI for performing various kinds of processing on a thumbnail. The window 60 is displayed on the display unit 12 when a predetermined item in the tool menu of the menu bar 58 in the window 50 (see FIG. 8) is selected. The display mode of the window 60 is controlled by the display controller 16a.

Specifically as shown in FIG. 9, the window 60 includes a maker appending area 61 which is for appending a desired mark to at least one thumbnail displayed in the subimage display area 53, a comment box 62 for displaying and creating a thumbnail comment to be appended to the thumbnail, and a dictionary 63 for inputting existing text information in the comment box 62.

The marker appending area 61 is a GUI for appending a desired mark to at least one thumbnail displayed in the subimage display area 53 in the window 50, and has substantially the same function as the marker appending area 45 in the window 40 (see FIG. 4). Specifically, the marker appending area 61 has a marker icon 61a for appending a marker having a circle shape or an oval shape, and a marker icon 61b for appending a marker having an arrow shape. As shown in FIG. 9, the marker appending area 61 displays a thumbnail selected by the user from at least one thumbnail in the subimage display area 53. The marker appending area 61 appends the marker having a circle shape or an oval shape on the displayed thumbnail when the marker icon 61a is clicked, and appends the marker having an arrow shape on the displayed thumbnail when the marker icon 61b is clicked.

Further, the marker appending area 61 has an "Undo" icon 61c for cancelling the marker appended on the thumbnail. The marker appending area 61 deletes various markers, i.e., markers of a circle shape, and oval shape, or an arrow shape, appended on the thumbnail when the "Undo" icon 61c is clicked.

The comment box 62 is a text box for displaying and creating a thumbnail comment of at least one thumbnail displayed in the subimage display area 53 in the window 50. Specifically, when the user selects a desired thumbnail from at least one thumbnail displayed in the subimage display area 53, the comment box 62 displays the thumbnail comment of the selected thumbnail in text data format. The thumbnail comment displayed in the comment box 62 can be changed to a desired thumbnail comment by operating the input unit 11. The comment processor 16c appends the thumbnail comment which has been changed in the comment box 62 to the selected desired thumbnail.

On the other hand, when no thumbnail comment is appended to the thumbnail, the comment box 62 is in a state of being ready for creating a new thumbnail comment, i.e., in a state of being blank. In this case, a desired thumbnail comment is input in the comment box 62 via the input operation of the input unit 11. The comment processor 16c appends the thumbnail comment input in the comment box 62 to the selected desired thumbnail.

The dictionary 63 has plural pieces of preset text data, and functions as a dictionary unit which retrieves and displays a candidate of text data to be displayed in the comment box 62 from the plural pieces of text data. Specifically, when a desired character such as an alphabet is input in the comment box 62, the dictionary 63 retrieves at least one piece of text data whose initial is the input character from the plural pieces of preset text data, and displays the retrieval result. Then, the dictionary 63 displays, in the comment box 62, text data selected by the user from at least one piece of text data displayed as the retrieval result. For example when the character "b" is input in the comment box 62, the dictionary 63 displays text data "blood" whose initial is the character "b" as a candidate. When the user selects the text data "blood", the dictionary 63 displays the selected text data "blood" in the comment box 62 as shown in FIG. 9. The dictionary function of the dictionary 63 can be switched over between a valid state and an invalid state via clicking on a check box 63a.

The window 60 is additionally displayed on the display unit 12 in a state where the window 50 displaying the intra-subject images of the subject 1 is open. Therefore, by using the window 60, the user can easily perform various kinds of processing such as a processing for appending a thumbnail comment and a processing for appending various markers on the thumbnail, without switching the window 50 to the window 40 (see FIG. 4).

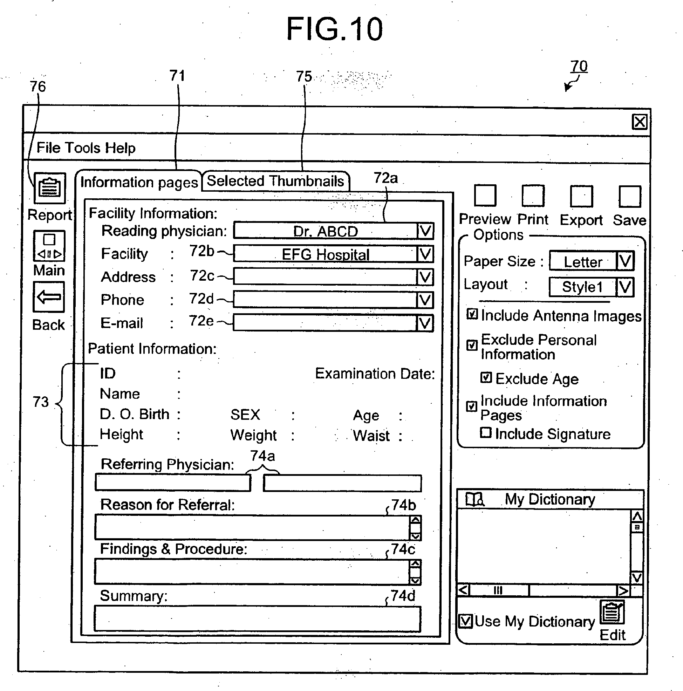

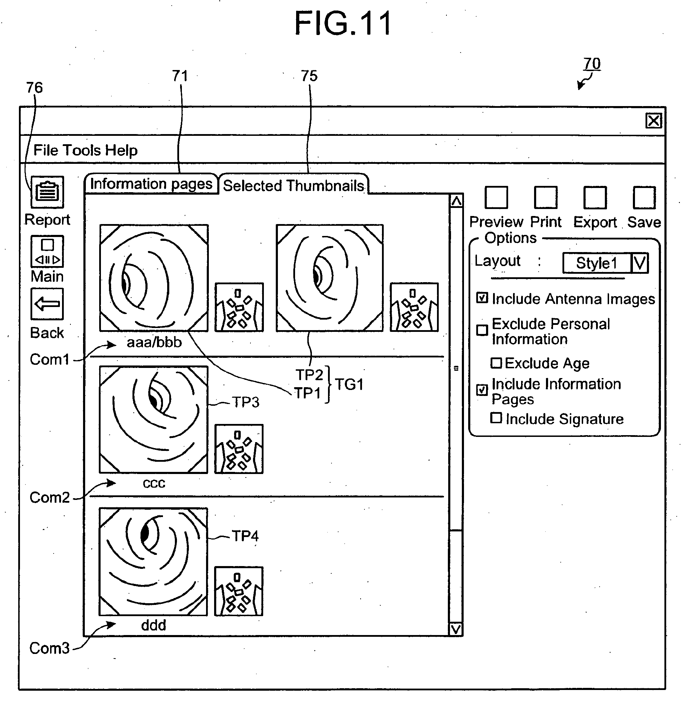

Next, a display mode of the display unit 12 in creating a diagnosis report of the subject 1 will be explained. FIG. 10 is a schematic view of an example of a window displayed on the display unit 12 in inputting various kinds of information such as a diagnosis result of the subject to be inserted to the diagnosis report. FIG. 11 is a schematic view of an example of a window displayed on the display unit 12 in checking various kinds of information such as a thumbnail and a thumbnail comment to be inserted to the diagnosis report. As shown in FIGS. 10 and 11, a window 70 has a GUI for creating a diagnosis report of the subject 1. The window 70 is displayed on the display unit 12 when a predetermined item in the tool menu of the menu bar 58 in the window 50 (see FIG. 8) is selected. The display mode of the window 70 is controlled by the display controller 16a.

Specifically as shown in FIGS. 10 and 11, the window 70 includes an information page 71 for editing various kinds of information such as the diagnosis result of the subject 1 to be inserted to the diagnosis report, a thumbnail page 75 for checking the thumbnail and the like to be inserted to the diagnosis report of the subject 1, and a report icon 76 for instructing to create the diagnosis report. The information page 71 and the thumbnail page 75 are displayed so as to be selective therebetween in the window 70. The information page 71 will be explained first, and then the thumbnail page 75 will be explained below.

As shown in FIG. 10, the information page 71 includes a doctor name setting area 72a for setting a doctor name to be inserted to the diagnosis report, i.e., a name of the doctor who made the diagnosis on the subject 1, a facility name setting area 72b for setting a facility name to be inserted to the diagnosis report, i.e., a name of the facility where the diagnosis on the subject 1 is made, an address setting area 72c for setting an address to be inserted to the diagnosis report, a telephone number setting area 72d for setting a telephone number to be inserted to the diagnosis report, and a mail address setting area 72e for setting an electronic mail address to be inserted to the diagnosis report. The information page 71 also includes an information display area 73 which displays patient information, examination information, and the like of the subject 1. The information page 71 further includes a text box 74a for inputting a doctor name, i.e., a name of the referral doctor to be inserted to the diagnosis report, a text box 74b for inputting a referral reason to be inserted to the diagnosis report, a text box 74c for inputting a diagnosis result and the like to be inserted to the diagnosis report, and a text box 74d for inputting a summary of the diagnosis result and the like to be inserted to the diagnosis report.

The doctor name setting area 72a functions as a text box capable of inputting text data via the operation of the input unit 11, and the name of the doctor and the like who made the diagnosis on the subject 1 is input therein. Further, the doctor name setting area 72a displays a drop-down menu of doctor name candidates via the operation of the input unit 11. The doctor name setting area 72a sets a desired doctor name selected by the user from the drop-down menu of the doctor name candidates as the doctor name to be inserted to the diagnosis report.

The facility name setting area 72b functions as a text box capable of inputting text data via the operation of the input unit 11, and the facility name such as a hospital and the like where the diagnosis on the subject 1 is made is input therein. Further, the facility name setting area 72b displays a drop-down menu of facility name candidates via the operation of the input unit 11. The facility name setting area 72b sets a desired facility name selected by the user from the drop-down menu of the facility name candidates as the facility name to be inserted to the diagnosis report.

The address setting area 72c functions as a text box capable of inputting text data via the operation of the input unit 11, and the address of the facility and the like where the diagnosis on the subject 1 is made is input therein. Further, the address setting area 72c displays a drop-down menu of address candidates via the operation of the input unit 11. The address setting area 72c sets a desired address selected by the user from the drop-down menu of the address candidates as the address to be inserted to the diagnosis report.

The telephone number setting area 72d functions as a text box capable of inputting text data via the operation of the input unit 11, and the telephone number of the doctor and the like who made the diagnosis on the subject 1 is input therein. Further, the telephone number setting area 72d displays a drop-down menu of telephone number candidates via the operation of the input unit 11. The telephone number setting area 72d sets a desired telephone number selected by the user from the drop-down menu of the telephone number candidates as the telephone number to be inserted to the diagnosis report.

The mail address setting area 72e functions as a text box capable of inputting text data via the operation of the input unit 11, and the electronic mail address of the doctor and the like who made the diagnosis on the subject 1 is input therein. Further, the mail address setting area 72e displays a drop-down menu of electronic mail address candidates via the operation of the input unit 11. The mail address setting area 72e sets a desired electronic mail address selected by the user from the drop-down menu of the electronic mail address candidates as the electronic mail address to be inserted to the diagnosis report.

The information display area 73 is an area for displaying the patient information, examination information, and the like of the subject 1 which are related to the diagnosis result and the like to be inserted to the diagnosis report. Specifically, in the information display area 73, the patient ID, name, birth date, sex, age, height, weight, waist size, and the like of the subject 1 are displayed as the patient information of the subject 1, and the date, ID, and the like of the examination are displayed as the examination information of the subject 1. Personal information including the birth date, sex, age, height, weight, waist size, and the like of the subject 1 among the patient information of the subject 1 can be displayed or hidden based on a selection via clicking on a specified check box, for example.

The text box 74a is for inputting a doctor name via the operation of the input unit 11. For example, a name of the referral doctor is input in the text box 74a in text data format. The text box 74b is for inputting a referral reason and the like via the operation of the input unit 11. For example, the reason why the patient is referred to the doctor and the like are input in the text box 74b in text data format. The text box 74c is for inputting a diagnosis result and the like via the operation of the input unit 11. For example, the diagnosis result and the like of the subject 1 are input in the text box 74c in text data format. The text box 74d is for inputting a summary and the like via the operation of the input unit 11. For example, a summary, findings, and the like of the diagnosis result of the subject 1 are input in the text box 74d in text data format.

Next, the thumbnail page 75 in the window 70 will be explained. As shown in FIG. 11, the thumbnail page 75 displays image information (a thumbnail, a thumbnail group, and the like) and various kinds of comment (a thumbnail comment, a group comment, and the like) to be inserted to the diagnosis report. The thumbnail or thumbnail group displayed in the thumbnail page is equivalent to the thumbnail or the thumbnail group which is displayed in the second thumbnail display area 42 in the window 40 (see FIG. 4).

Specifically as shown in FIG. 4, when the thumbnail group TG1, and thumbnails TP3 and TP4 are displayed in the second thumbnail display area 42, the thumbnail page 75 displays the thumbnails TP1 and TP2 included in the thumbnail group TG1, the thumbnail TP3, and the thumbnail TP4 respectively in separate rows. In other words, the thumbnail page 75 displays the thumbnails TP1 and TP2 included in the thumbnail group TG1 in a first row, the thumbnail TP3 in a second row, and the thumbnail TP4 in a third row.

Each row in the thumbnail page has a comment display area which displays a thumbnail comment or a group comment. Specifically, a comment display area Com1 is formed in the first row where the thumbnail group TG1 (thumbnails TP1 and TP2) is displayed, a comment display area Com2 is formed in the second row where the thumbnail TP3 is displayed, and a comment display area Com3 is formed in the third row where the thumbnail TP4 is displayed. In this case, the group comment "aaa/bbb" of the thumbnail group TG1 is displayed in the comment display area Coml, the thumbnail comment "ccc" of the thumbnail TP3 is displayed in the comment display area Com2, and the thumbnail comment "ddd" of the thumbnail TP4 is displayed in the comment display area Com3.

By displaying the thumbnail page 75 on the display unit 12, the user can check the image content and the sequence of the thumbnail and the thumbnail group to be inserted to the diagnosis report of the subject 1, and the content and the sequence of each comment of the thumbnail and the thumbnail group (a thumbnail comment and a group comment).

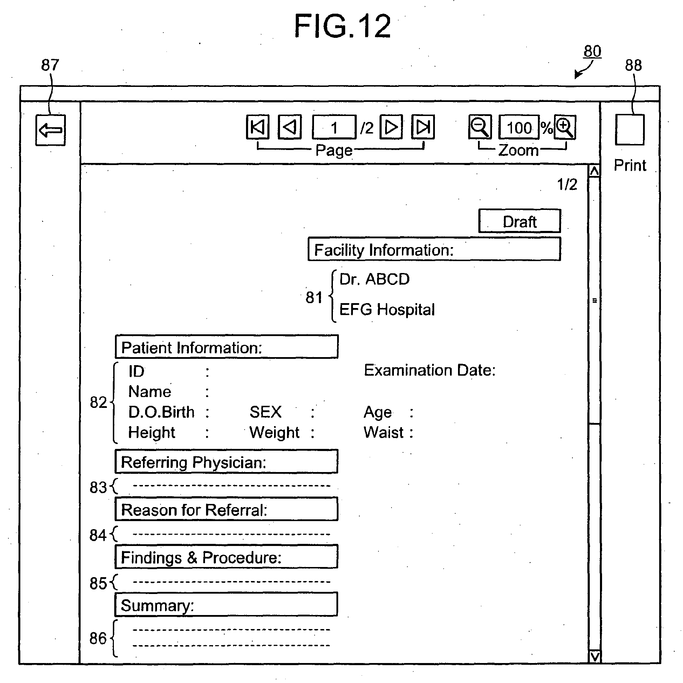

Next, a display mode of the display unit 12 in displaying the diagnosis report of the subject 1 created by the report processor 16d, and an operation of the report processor 16d which creates the diagnosis report of the subject 1 will be explained. FIG. 12 is a schematic view of an example of a window displaying the diagnosis report of the subject 1. As shown in FIG. 12, a window 80 has a GUI for displaying or printing the diagnosis report of the subject 1 created by the report processor 16d. The window 80 is displayed on the display unit 12 when the report icon 76 (see FIGS. 10 and 11) in the window 70 is clicked, for example. The display mode of the window 80 is controlled by the display controller 16a.

Specifically as shown in FIG. 12, the window 80 displays the diagnosis report (draft) of the subject 1 created by the report processor 16d. In the diagnosis report displayed in the window 80, a plurality of information insertion columns 81 to 86 are formed respectively in separate rows. The window 80 includes a "Back" icon 87 for re-creating the diagnosis report and a "Print" icon 88 for printing the diagnosis report.

When the "Report" icon 76 in the window 70 is clicked, the input unit 11 inputs information instructing to create the diagnosis report to the control unit 16. In this case, the report processor 16d acquires, based on the instruction to create the diagnosis report, information of the subject 1 which is input (created) in the information page 71 of the window 70, and comment information such as image information of the thumbnail displayed in the thumbnail page and the thumbnail comment, and creates the diagnosis report of the subject 1 based on various kinds of acquired information.

Specifically, the report processor 16d uses at least one of the doctor name set in the doctor name setting area 72a, the facility name set in the facility name setting area 72b, the address set in the address setting area 72c, the telephone number set in the telephone number setting area 72d, and the electronic mail address set in the mail address setting area 72e to create facility information to be inserted to the diagnosis report. In other words, the report processor 16d uses only information set (input in text data format) in the information page 71 among the doctor name, facility name, address, telephone number, and electronic mail address to create the facility information. The report processor 16d inserts the facility information to the information insertion column 81 of the diagnosis report (draft).

For example, when a doctor name "Dr. ABCD" and a facility name "EFG Hospital" are set in the doctor name setting area 72a and the facility name setting area 72b, respectively, and no text data for the address, telephone number, and electronic mail address is set in the address setting area 72c, telephone number setting area 72d, and mail address setting area 72e, respectively, the report processor 16d uses only text data for the doctor name "Dr. ABCD" and the facility name "EFG Hospital" set in the doctor name setting area 72a and the facility name setting area 72b, respectively to create the facility information. In this case, the report processor 16d inserts only the doctor name "Dr. ABCD" and the facility name "EFG Hospital" in the information insertion column 81 as shown in FIG. 12.

Further, the report processor 16d inserts various kinds of information (the patient information, examination information, and the like of the subject 1) which is displayed in the information display area 73 of the information page 71 into the information insertion column 82 of the diagnosis report (draft). Furthermore, the report processor 16d inserts text data (doctor name) input in the text box 74a into the information insertion column 83, text data (referral reason and the like) input in the text box 74b into the information insertion column 84, text data (diagnosis result and the like of the subject 1) input in the text box 74c into the information insertion column 85, and text data (summary and the like) input in the text box 74d into the information insertion column 86.

Moreover, the report processor 16d inserts image information (thumbnail, thumbnail group, and the like) and comment information (thumbnail comment and group comment) displayed in the thumbnail page 75 into a page (not shown) on and after the second page of the diagnosis report (draft) of the subject 1. In this case, the report processor 16d arranges the image information such as the thumbnail in the diagnosis report and inserts comment information to a position adjacent to the image information, in a similar way to the display mode of the thumbnail page 75 (see FIG. 11).

The report processor 16d creates a diagnosis report page (first page) shown in FIG. 12 and a diagnosis report page (second page) whose display mode is similar to the thumbnail page 75 shown in FIG. 11. The diagnosis report of the subject 1 created by the report processor 16d is output in print via the output unit 14 when the "Print" icon 88 is clicked. On the other hand, when the "Back" icon 87 is clicked, the display unit 12 displays the window 70 in place of the window 80. In other words, the display unit 12 makes the display screen of the diagnosis report (draft) back to a screen for creating the diagnosis report.

In the embodiment of the present invention, the comment processor 16c divides, when the thumbnail group is ungrouped, the group comment of the thumbnail group to a thumbnail or a thumbnail group. However the present invention is not limited to this configuration, and the comment processor 16c may not change (divide) the group comment of the thumbnail group even when the thumbnail group is ungrouped. In this case, the group comment of the ungrouped thumbnail group may be changed by using the comment box 43.

When a group comment is to be newly created, the comment processor 16c may combine the thumbnail comment or the group comment along the sequence of at least one thumbnail or at least one thumbnail group included in the thumbnail group corresponding to the new group comment.
The comment processor 16c may, otherwise, combine the thumbnail comment or the group comment along a time-series sequence (a sequence of imaging times of thumbnails, for example).

As described above, in the embodiment of the present invention, the image display apparatus is configured to include the grouping processor that groups a plurality of thumbnails corresponding to a plurality of intra-subject images selected from the intra-subject images of the subject, respectively, and a comment processor that performs various kinds of processing about the thumbnail comment of the thumbnail. In the image display apparatus, the comment processor appends thumbnail comments respectively to the plurality of thumbnails, and when the grouping processor creates a thumbnail group where the plurality of thumbnails area grouped, the comment processor combines thumbnail comments respectively of the plurality of thumbnails included in the thumbnail group to create a group comment of the thumbnail group. Therefore in the image display apparatus, even though the user does not manually input the group comment by operating the input unit, a group comment can automatically be created by making good use of thumbnail comments respectively of the plural thumbnails included in the thumbnail group. As a result, a cumbersome of the manual operation performed by the user can be reduced and the image display apparatus capable of easily creating a group comment for each thumbnail group can be realized.

In addition, the comment processor puts a predetermined separator or a space (blank) between respective thumbnail comments constituting the group comment, and combines the plurality of thumbnail comments in a state where respective thumbnails can be discriminated to each other. Therefore, by visually checking the group comment of the thumbnail group, each thumbnail comment of the plurality of thumbnails included in the thumbnail group can be easily discriminated.

Further, when the grouping processor ungroups the thumbnail group, the comment processor divides the group comment of the ungrouped thumbnail group with respect to each thumbnail or thumbnail group. Therefore, comment information (thumbnail comment and group comment) of at least one thumbnail or at least one thumbnail group divided (excluded) from the thumbnail group as an ungrouping target can be easily set.

In the image display apparatus according to the present invention, when a thumbnail group in which a plurality of thumbnails are grouped is created, thumbnail comments respectively of the plurality of thumbnails included in the thumbnail group are combined to create a group comment of the thumbnail group. Therefore, a group comment can be automatically created by using thumbnail comments respectively of the plurality of thumbnails included in the thumbnail group even though the user does not perform the input operation of the group comment whenever a thumbnail group is created. As a result, the cumbersome of the manual operation performed by the user can be reduced, and a group comment of each thumbnail group can be easily created as advantages of the present invention.

As described above, the image display apparatus according to the present invention is useful for an examination of an inside of a subject such as a patient via an observation of images capturing the inside of organs of the subject, and specifically suitable as an image display apparatus which can easily create a desired group comment with respect to each thumbnail group in which a plurality of thumbnails corresponding to intra-subject images are grouped.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. An image display apparatus (4) comprising:
a display unit (12) that displays a series of intra-subject images capturing an inside of a subject (1) along time series, and additionally displays a thumbnail corresponding to an intra-subject image selected from the series of intra-subject images;
a grouping processor (16b) that creates a thumbnail group including a plurality of thumbnails;
an input unit (11) that inputs a thumbnail comment about the thumbnail; and
a comment processor (16c) that appends the thumbnail comment to the thumbnail, combines a plurality of thumbnail comments respectively of the plurality of thumbnails included in the thumbnail group whenever the grouping processor (16b) creates the thumbnail group, and creates a group comment of the thumbnail group.

2. The image display apparatus (4) according to claim 1, wherein
the grouping processor (16b) excludes at least one thumbnail from the thumbnail group and changes the thumbnail group to a different thumbnail group, and
the comment processor (16c) deletes at least one thumbnail comment of the at least one thumbnail from the group comment of the thumbnail group, creates a different group comment of the different thumbnail group, and appends the at least one deleted thumbnail comment to the at least one excluded thumbnail.

3. The image display apparatus (4) according to claim 2, wherein
the grouping processor (16b) excludes a plurality of thumbnails from the thumbnail group, changes the thumbnail group to the different thumbnail group, and further creates another different thumbnail group including the plurality of thumbnails excluded from the thumbnail group, and
the comment processor (16c) deletes a plurality of thumbnail comments respectively of the plurality of thumbnails in the another different thumbnail group from the group comment of the thumbnail group, creates the different thumbnail comment of the different thumbnail group, combines the plurality of deleted thumbnail comments, and creates another different group comment of the another different thumbnail group.

4. The image display apparatus (4) according to claim 1, wherein
the grouping processor (16b) combines the thumbnail group including the plurality of thumbnails and at least one thumbnail to create a different thumbnail group, and the comment processor (16c) combines the group comment of the thumbnail group and at least one thumbnail comment of the at least one thumbnail, and creates a different group comment of the different thumbnail group.

5. The image display apparatus (4) according to claim 1, wherein
the grouping processor (16b) combines a plurality of thumbnail groups to create a different thumbnail group, and
the comment processor (16c) combines a plurality of group comments respectively of the plurality of thumbnail groups, and creates a different group comment of the different thumbnail group.

6. The image display apparatus (4) according to claim 1, wherein the comment processor (16c) combines the plurality of thumbnail comments so that the respective thumbnail comments can be discriminated to each other, and creates the group comment of the thumbnail group.

7. The image display apparatus (4) according to claim 1, further comprising a report creating unit (16d) that creates a diagnosis report of the subject (1), the diagnosis report inserting therein the thumbnail group and the group comment of the thumbnail group.
